# EUROPEAN PATENT SPECIFICATION

(11) **EP 4 734 850 B1**
(45) Date of publication and mention of the grant of the patent: **14.10.2026**
(21) Application number: 24735204.0
(22) Date of filing: 19.06.2024
(51) Int. Cl.: A61B 6/00, A61B 5/00, G01R 33/48

(54) **MEDICAL SCANNER**
MEDIZINISCHER SCANNER
SCANNER MÉDICAL

(30) Priority: 27.06.2023 EP 23181714
(43) Date of publication of application: 06.05.2026
(73) Proprietor: Koninklijke Philips N.V., 5656 AE Eindhoven (NL)
(72) Inventor: MOTIYAR, Rahul, 5656 AG Eindhoven (NL); KNOESTER, Jaap, 5656 AG Eindhoven (NL); SENEGAS, Julien Thomas, 5656 AG Eindhoven (NL); WEISS, Steffen, 5656 AG Eindhoven (NL)
(74) Representative: Philips Intellectual Property & Standards
(86) International application number: PCT/EP2024/067021
(87) International publication number: WO 2025/002929

(56) References cited:
- JP-A- 2009 119 281
- US-A1- 2020 205 748

## Description

### FIELD OF THE INVENTION

This invention relates to medical scanners, and in particular relates to detection of the Valsalva maneuver during a scan.

### BACKGROUND OF THE INVENTION

The primary cause for motion artefacts in radiology exams, such as CT scanning using a contrast agent, is the Valsalva maneuver. The Valsalva maneuver is one important factor which causes a transient interruption of contrast (TIC) in the veins and arteries that leads to the need for a repetition of scans or re-injection of the contrast agent. Reappointments will even be needed if there is already enough contrast agent present in the body.

The Valsalva maneuver is the forced attempt to expire air against a closed airway, resulting in increased intra-abdominal, intrathoracic, and pharyngeal pressure. This phenomenon of sudden fixating of breathing and tensioning of the chest muscles causes a contrast agent to slow down in the first few seconds and then move too quickly once the a sympathetic response is triggered to loosen up the heart muscles, and blood flow increases. The contrast agent may then be unable to reach the location at the desired time and the radiographer and scanner may then miss the scanning window.

This transient interruption of contrast is a common phenomenon in CT pulmonary angiography (CTPA) studies. The contrast opacification of the pulmonary arteries is suboptimal due to an increase in the flow of un-opacified blood from the inferior vena cava (IVC) to the right side of the heart, often during deep inspiration.

There are several types of CT exams which are supposed to be performed during a breath hold and without the Valsalva maneuver, such as chest and abdomen CT scans, and scans for cardiac and pulmonary embolism cases.

There are also some specific exams which, if conducted during a Valsalva maneuver, can given improved detection and better characterization of anatomy, such as a CT scan of a Hernia, or an ultrasound scan for Vena Cava function.

Thus, it is desirable to be able to detect the presence of the Valsalva maneuver, to confirm its presence if it is desired, or take remedial action when it is not desired.

US 2020/205748 A1 describes a system for detecting patient motion during a medical imaging session, and adjusting one or more aspects of the session based on that motion.

JP 2009 119281 A describes a setup comprising a video camera for continuously acquiring a medical image of a test subject and a console that displays the video to an operator.

### SUMMARY OF THE INVENTION

The invention is defined by the claims.

According to examples in accordance with an aspect of the invention, there is provided a medical scanner, comprising:
a scanning system;
a detection system for generating a detection output; and
a processor configured to:
   interpret the detection output thereby to detect a Valsalva maneuver when present; and
   provide output instructions advising to cease the delivery of scanning radiation or to cease contrast injection, or control the medical scanner to cease the delivery of scanning radiation or cease contrast injection, in response to detection of a Valsalva maneuver.

This system enables detection of a Valsalva maneuver, and takes appropriate action when detected. At least in some situations, this involves ceasing contrast injection or ceasing imaging (or providing instructions to the operator to do so). In some other situations, it may be appropriate to provide user advice or training.

For those CT exams which are supposed to be done during a breath hold but without the Valsalva maneuver, when a Valsalva maneuver is detected, the scan should be stopped and no further contrast agent should be added (so that another scan can be performed without delivering more than a desired amount of contrast agent).

In other situations, the Valsalva maneuver may be desired, to help in the detection and better characterization of the anatomy such as for a CT scan of a hernia, or an ultrasound scan for Vena Cava function etc. In those situations, the detection of the Valsalva Maneuver may be detected to confirm its presence.

Thus, while one primary use of the system is to cease radiation exposure and contrast injection when the Valsalva maneuver is detected, the detection may be used for other purposes in other use cases of the medical scanner.

The detection system for example comprises an in-bore camera. This provides a low cost solution.

The processor arrangement may then be configured to interpret signals generated by the in-bore camera to detect one or more of:
respiration rate;
heart rate;
blood pressure;
facial expression; and
skin tone variation.

Thus, the camera may be used to detect various vital signs, and one or more of these may be used to detect a Valsalva maneuver.

For example, in a first arrangement the processor arrangement may be configured to detect a Valsalva maneuver based on a change of skin tone from a baseline skin tone.

In a second arrangement, the processor arrangement may be configured to detect a Valsalva Maneuver based on a drop of PPG amplitude from a remote PPG signal.

In one possible use case, the processor arrangement may be configured to interpret the detection output thereby to detect Valsalva maneuver during a surview, and when detected to generate advisory information for a subsequent clinical scan.

In another possible use case, the processor arrangement may be configured to cease delivery of scanning radiation or provide instructions for the operator to end the delivery of scanning radiation if a Valsalva maneuver is detected during a contrast scan. The contrast scan may be timed or non-timed or a bolus tracking contrast scan.

In another possible use case, the processor arrangement may be configured to end contrast injection if Valsalva maneuver is detected during a breath hold of a contrast scan.

A display may be provided for presenting information to a subject while scanning takes place, wherein the processor arrangement is further configured to control the display to present breath hold instructions to the subject. The same display may also be used for providing the advisory or educational messages to the subject as discussed above.

In some examples, the scanning system is a CT scanning system. In other examples, the scanning system is a MRI scanning system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:

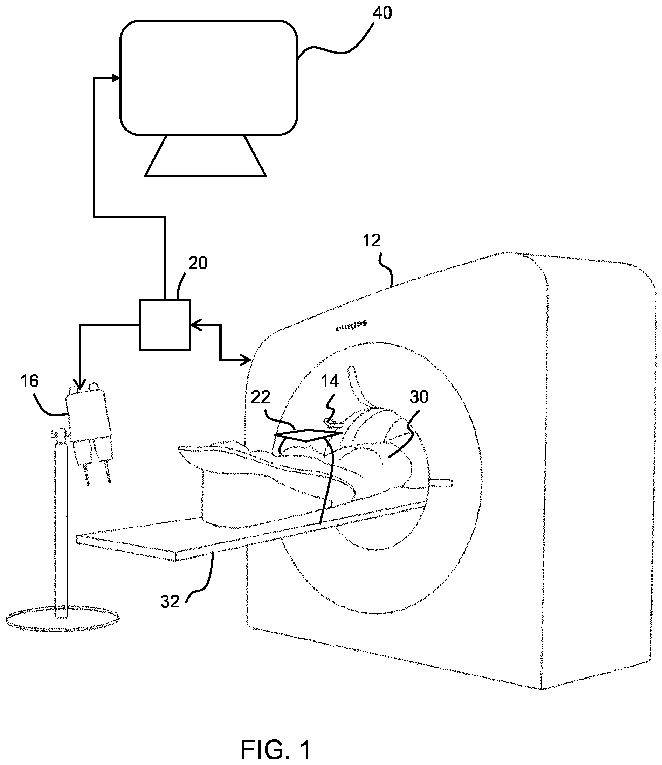

Fig. 1 shows a medical scanner.

### DETAILED DESCRIPTION OF THE EMBODIMENTS

The invention will be described with reference to the Figure.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figure is merely schematic and are not drawn to scale. It should also be understood that the same reference numerals are used throughout the Figure to indicate the same or similar parts.

The invention provides a medical scanner that has a detection system to enable a Valsalva maneuver to be identified. The scanner is controlled to cease the delivery of scanning radiation or cease contrast injection, or else user advice is provided to cease the delivery of scanning radiation or cease contrast injection, in response to detection of a Valsalva maneuver.

Fig. 1 shows a medical scanner 10 comprising a scanning system 12 such as a CT bore and a detection system 14 for generating a detection output which can be interpreted to detect a Valsalva maneuver when present. The detection system in preferred examples comprises an in-bore camera.

The scanner also has a contrast injection system 16 for injecting a contrast agent for contrast scans.

A processor arrangement 20 is shown schematically in Fig. 1, for interpreting the detection output to detect a Valsalva maneuver and optionally also controlling the scanning system and injection system.

A system is provided for presenting instructions or advice to the patient during the scan, such as a color display panel 22 mounted inside the bore and associated speaker.

The scanner has a couch 32 that moves through the bore, and a patient 30 is shown on the couch. A display console 40 is also shown for the scanning system operator (i.e., user).

For the detection of the Valsalva maneuver, various vital signs and other patient information may be measured and analyzed, including one or more of the following:
Heart Rate (using remote PPG implemented using the camera)
Blood Pressure (using remote PPG implemented using the camera)
Muscle tension (using galvanic skin response electrodes)
Measuring skin tone variation (using remote PPG or camera image analysis)
Facial expression (using camera image analysis).

One specific example of camera-based detection of Valsalva can be based on a change of skin tone. A Valsalva maneuver causes the blood content of the skin to change, which is observable by an RGB camera as a change in the DC level of the skin color values. A baseline skin color is monitored, and the onset of Valsalva can be detected as a deviation from this baseline color by a pre-set threshold value. This threshold is selected based on a large cohort of volunteers and may be set depending on skin type, age, and gender.

Another specific of camera-based detection can be based on a remote PPG signal A sudden decrease of the PPG amplitude by typically 40% or more at the onset of Valsalva may be exploited to detect Valsalva.

A typical CT exam involves a surview, which is a low dose trial exposure to identify the area of interest for the clinical scan, and one or many clinical scans with or without the use of a contrast agent.

For a breath-hold scan the patients are supposed to perform an inhalation and then hold their breath upon receiving a breath hold instruction (e.g. via the display panel or by audio instruction). The breath hold is critical for the clinical scans, whereas the surview is used to educate the patient in practicing the breath-hold for the subsequent clinical scan, which is to be used for interpretation purposes.

The display 22 is used to guide the patient throughout the exam to enable anticipation of breath hold and to instruct the start and stop (and duration) of the breath hold.

When the patient 30 lies on the couch 32, the in-bore camera 14 starts monitoring the respiration rate, heart rate, blood pressure, facial expression and skin tone variation. A separate galvanic skin response sensor may be used to monitor muscle tension. If a Valsalva maneuver is detected then the operator is informed via the console software with a suggestion for optimizing the scan. In some examples, there may also be automatic control of the scanning process in response to the detection.

Different automatic actions or suggestions will be appropriate for different types of scan, and for the surview compared to the clinical scan. Examples are discussed further below.

In general, if Valsalva is detected during surview, then after the surview the operator is informed about the Valsalva incident and necessary suggestions are provided for the next scan. Optimization suggestion could involve guiding the patient on breathing technique, changing the language of software generated instructions if the patient is unable to understand the language, changing the patient orientation if that helps the patient, etc.

During a non-timed or timed contrast clinical scan, If Valsalva is detected then radiation should be stopped, either automatically or by informing the operator to stop the scan to avoid unnecessary exposure.

During a bolus tracking-contrast clinical scan, if Valsalva is detected then radiation may again be immediately stopped or the operator may be informed to stop the scan. If the contrast is half-way delivered, contrast administration injection may also be stopped and saline can be given to flush out the contrast from anatomy. If contrast is already completely delivered, more saline can be injected to flush the contrast from anatomy.

As mentioned above, there are other scan scenarios where a Valsalva maneuver is desired as it can improve the image quality. In such cases, the aim is to instruct the patient to be able to perform the Valsalva maneuver at the desired time.

Some possible use cases will be set out below in more detail, together with an explanation of what action is taken when a Valsalva maneuver is detected but it is not desired, or when a Valsalva maneuver is not detected but it is desired.

A first category is for exams which are expected to be free of Valsalva. Indeed, the majority of CT breath-hold exams are expected to be free of Valsalva so that contrast can flows evenly and gradually. This for example applies to scans of the lungs, cardiac or abdomen scans. In such cases, the following actions can be taken:

### A. Non-contrast scan

### (i) Surview

If Valsalva is detected in the surview, the operator is informed during or after the surview is finished so that the patient can be coached for correct breath-hold performance so that Valsalva is avoided for the subsequent clinical scan. Surviews are not impacted by Valsalva, so no retake of the surview is required.

### (ii) Clinical scan

If Valsalva is detected during the clinical scan, and the image quality is impacted, then retakes is suggested by system.

### B. Timed contrast scan

### (i) Surview

If Valsalva is detected in the surview, the operator is informed during or after the surview is finished so that the patient can be coached for breath-hold again avoiding Valsalva for the clinical scan. If needed, a demo acquisition (without radiation) can be done to ensure the patient has understood how the breath-hold needs to be done.

### (ii) Clinical scan

If Valsalva is detected during the acquisition then the patient can be guided during acquisition, or the acquisition can be stopped immediately to save the radiation dose and a retake can be performed on time while the contrast is still present in the body. Alternatively, acquisition can be stopped immediately to save the radiation dose and a retake can be performed after re-administrating contrast and guiding the patient again.

If Valsalva is detected post scan and the image quality is impacted then parameters suggesting retake can be shown immediately after the clinical scan (before final reconstruction takes place in order to save time).

### C. Bolus tracking

### (i) Surview

If Valsalva is detected in the surview, the operator is informed during or after the surview is finished so that the patient can be coached for breath-hold again avoiding Valsalva for the clinical scan. If needed, a demo acquisition (without radiation) can be done to ensure the patient has understood how the breath-hold needs to be done.

### (ii) Clinical scan

If Valsalva is detected during the acquisition then the patient can be guided during acquisition or acquisition and contrast administration can be stopped immediately to save radiation dose and retake can be performed on time by re-administrating the contrast and guiding the patient again.

If Valsalva is detected post scan and the image quality is impacted then parameters suggesting retake can be shown immediately after the clinical scan (before final reconstruction takes place in order to save time). If a retake is not possible for example for brain perfusion scanning, where the timing of the contrast is a key element, then information of "Valsalva detected during clinical scan" may be tagged along with the image to ensure a radiologist reads the image accordingly and provides appropriate conclusions.

A second category is for exams for which Valsalva is needed. Certain CT breath-hold exams are expected to be with Valsalva so that better characteristics of the inner walls of organs can be seen. This for example applies to scans of a hernia, the cheeks/oral cavity or for heart failure. In such cases, the following actions can be taken:

### D. Non-contrast scan

### (i) Surview

If Valsalva is not detected in the surview, then the patient is coached again for the clinical scan.

### (ii) Post scan

If Valsalva is not present and the final construction is not satisfactory (i.e., doesn't produce enough characteristic differences in tissues), a retake is suggested with the system suggesting changes to relevant parameters for the clinical scan such as longer scan times, slowing rotation speed. The patient can also be coached again for the clinical scan.

### E. Timed contrast scan

### (i) Surview

If Valsalva is not detected in the surview, then the patient is coached again for the clinical scan.

### (ii) Post scan

If Valsalva is not present and the final construction is not satisfactory (i.e., doesn't produces enough characteristic differences in tissues), a retake is suggested with the system suggesting change relevant parameters for the clinical scan such as longer scan times, slowing rotation speed. The patient can also be coached again for the clinical scan.

### C. Bolus tracking

### (i) Surview

If Valsalva is not detected in the surview, then the patient is coached again for the clinical scan.

### (ii) Post scan

If Valsalva is not achieved during first few seconds then acquisition and contrast administration can be stopped immediately to save radiation dose and a retake can be performed on time by re-administrating the contrast and guiding the patient again. If a retake is not possible then the system may suggest post processing methods by which tissue characteristics can be improved.

These examples relate to different types of CT scan. However, the invention may also be applied to other imaging types.

For example, Valsalva is used also in MRI similarly to CT to better depict certain pathologies, mainly of the pelvic floor and anal canal, but also in the head and neck region. Mostly, patients perform the Valsalva on command and without any monitoring, but relying on the patient to comply. Thus, the approach of the invention can again be used to coach patient to perform the Valsalva maneuver when it is detected that it is not present, or propose retake of scans.

In other, breath-hold, MRI exams, spontaneous Valsalva during the scan impairs image quality in e.g., cardiac and brain imaging, generally in all methods which require breath-holds and are very sensitive to motion. Many MR scans consist of multiple breath-holds. In the case of a spontaneous Valsalva during one of the breath-holds, this condition can be detected so that the breath hold can be interrupted. The patient can then breathe, and then the scan can be continued with the next breath-hold. If a spontaneous Valsalva affects only a part of the breath-hold, the other part of the data may be used and corrupted data can be reacquired in the next breath-hold. In the case of detection of spontaneous Valsalva maneuvers in a particular patient, the MR system may change to, or propose, a different kind of scan or adapt the parameters of the planned scan with the aim to make it less sensitive to motion artifacts. Details of these adaptations are known and are dependent on the type of scan.

It can be seen from the examples above than the detection of the presence of, or the absence of, Valsalva has potential applications in a variety of types of scan.

As will also be clear from the above, the core concept of the invention is to instruct or control the ceasing of scanning radiation or the ceasing of contrast injection in response to detection of a Valsalva maneuver. The system of the invention may offer other options in response to detection of Valsalva, so that it can provide appropriate action for a variety of different scenarios, as explained above.

Variations to the disclosed embodiments can be understood and effected by those skilled in the art in practicing the claimed invention, from a study of the drawings, the disclosure and the appended claims. **In** the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A medical scanner (10), comprising:
a scanning system (12);
a detection system (14) for generating a detection output; and
a processor (20) configured to:
interpret the detection output thereby to detect a Valsalva maneuver when present; and
provide output instructions advising to cease the delivery of scanning radiation or to cease contrast injection, or control the medical scanner to cease the delivery of scanning radiation or cease contrast injection, in response to detection of a Valsalva maneuver.

2. The medical scanner of claim 1, wherein the detection system comprises an in-bore camera (14).

3. The medical scanner of claim 2, wherein the processor arrangement (20) is configured to interpret signals generated by the in-bore camera to detect one or more of:
respiration rate;
heart rate;
blood pressure;
facial expression; and
skin tone variation.

4. The medical scanner of claim 3, wherein the processor arrangement (20) is configured to detect a Valsalva maneuver based on a change of skin tone from a baseline skin tone.

5. The medical scanner claim 3 or 4, wherein the processor arrangement (20) is configured to detect a Valsalva Maneuver based on a drop of PPG amplitude from a remote PPG signal.

6. The medical scanner of any one of claims 1 to 5, wherein the processor arrangement (20) is configured to interpret the detection output thereby to detect Valsalva maneuver during a surview, and when detected to generate advisory information for a subsequent clinical scan.

7. The medical scanner of any one of claims 1 to 5, wherein the processor arrangement (20) is configured to cease delivery of scanning radiation or provide instructions for a scan operator to end the delivery of scanning radiation if a Valsalva maneuver is detected during a contrast scan.

8. The medical scanner of any one of claims 1 to 5, wherein the processor arrangement (20) is configured to end contrast injection if Valsalva maneuver is detected during a breath hold of a contrast scan.

9. The medical scanner of any one of claims 1 to 8, further comprising a display (40) for presenting information to a subject while scanning takes place, wherein the processor arrangement is further configured to control the display to present breath hold instructions to the subject.

10. The medical scanner of any one of claims 1 to 9, wherein the scanning system (12) is a CT scanning system.

11. The medical scanner of any one of claims 1 to 9, wherein the scanning system (12) is a MRI scanning system.

## Patentansprüche

1. Medizinischer Scanner (10), umfassend:
ein Scansystem (12);
ein Detektionssystem (14) zum Erzeugen einer Detektionsausgabe; und
einen Prozessor (20), der so konfiguriert ist, dass er:
die Detektionsausgabe interpretiert, um dadurch ein Valsalva-Manöver zu erkennen, wenn ein solches vorliegt; und
Ausgabeanweisungen bereitstellt, die dazu auffordern, die Abgabe von Scanstrahlung zu stoppen oder Kontrastmittelinjektion zu stoppen, oder den medizinischen Scanner so steuert, dass er in Reaktion auf die Erkennung eines Valsalva-Manövers die Abgabe von Scanstrahlung stoppt oder Kontrastmittelinjektion stoppt.

2. Medizinischer Scanner nach Anspruch 1, wobei das Detektionssystem eine In-Bore-Kamera (14) umfasst.

3. Medizinischer Scanner nach Anspruch 2, wobei die Prozessorbaugruppe (20) so konfiguriert ist, dass sie von der In-Bore-Kamera erzeugte Signale interpretiert, um eines oder mehrere zu erkennen von:
Atemfrequenz;
Herzfrequenz;
Blutdruck;
Gesichtsausdruck; und
Variation des Hauttons.

4. Medizinischer Scanner nach Anspruch 3, wobei die Prozessorbaugruppe (20) so konfiguriert ist, dass sie ein Valsalva-Manöver auf Grundlage einer Veränderung des Hauttons gegenüber einem Ausgangshautton erkennt.

5. Medizinischer Scanner nach Anspruch 3 oder 4, wobei die Prozessorbaugruppe (20) so konfiguriert ist, dass sie ein Valsalva-Manöver auf Grundlage eines Abfalls der PPG-Amplitude gegenüber einem Remote-PPG-Signal erkennt.

6. Medizinischer Scanner nach einem der Ansprüche 1 bis 5, wobei die Prozessorbaugruppe (20) so konfiguriert ist, dass sie die Detektionsausgabe interpretiert, um dadurch ein Valsalva-Manöver während einer Untersuchung zu detektieren, und wenn dieses erkannt wird, Ratschläge für einen anschließenden klinischen Scan erzeugt.

7. Medizinischer Scanner nach einem der Ansprüche 1 bis 5, wobei die Prozessorbaugruppe (20) so konfiguriert ist, dass sie die Abgabe von Scanstrahlung stoppt oder einem den Scan durchführenden Bediener Anweisungen bereitstellt, die Abgabe von Scanstrahlung zu beenden, wenn während eines Kontrastmittel-Scans ein Valsalva-Manöver erkannt wird.

8. Medizinischer Scanner nach einem der Ansprüche 1 bis 5, wobei die Prozessorbaugruppe (20) so konfiguriert ist, dass sie die Kontrastmittelinjektion beendet, wenn während des Anhaltens des Atems bei einem Kontrastmittel-Scan ein Valsalva-Manöver erkannt wird.

9. Medizinischer Scanner nach einem der Ansprüche 1 bis 8, ferner umfassend eine Anzeige (40) zum Anzeigen von Informationen für ein Subjekt, während der Scan läuft, wobei die Prozessorbaugruppe weiter so konfiguriert ist, dass sie die Anzeige so steuert, dass dem Subjekt Anweisungen zum Anhalten des Atems angezeigt werden.

10. Medizinischer Scanner nach einem der Ansprüche 1 bis 9, wobei es sich bei dem Scansystem (12) um ein CT-Scansystem handelt.

11. Medizinischer Scanner nach einem der Ansprüche 1 bis 9, wobei es sich bei dem Scansystem (12) um ein MRT-Scansystem handelt.

## Revendications

1. Scanner médical (10), comprenant :
un système de numérisation (12) ;
un système de détection (14) pour générer un signal de détection ; et
un processeur (20) configuré pour :
interpréter ainsi le résultat de la détection afin de détecter une manœuvre de Valsalva lorsqu'elle est présente ; et
fournir des instructions de sortie conseillant d'arrêter l'administration de rayonnements de balayage ou d'arrêter l'injection de produit de contraste, ou de commander le scanner médical pour arrêter l'administration de rayonnements de balayage ou arrêter l'injection de produit de contraste, en réponse à la détection d'une manœuvre de Valsalva.

2. Scanner médical selon la revendication 1, dans lequel le système de détection comprend une caméra interne (14).

3. Scanner médical selon la revendication 2, dans lequel l'agencement de processeur (20) est configuré pour interpréter des signaux générés par la caméra interne afin de détecter un ou plusieurs éléments parmi :
le rythme respiratoire ;
la fréquence cardiaque ;
la pression artérielle ;
l'expression faciale ; et
la variabilité du teint.

4. Scanner médical selon la revendication 3, dans lequel l'agencement de processeur (20) est configuré pour détecter une manœuvre de Valsalva basée sur un changement de teint de la peau par rapport à un teint de base.

5. Scanner médical selon la revendication 3 ou 4, dans lequel l'agencement de processeur (20) est configuré pour détecter une manœuvre de Valsalva basée sur une chute d'amplitude PPG à partir d'un signal PPG distant.

6. Scanner médical selon l'une quelconque des revendications 1 à 5, dans lequel l'agencement de processeur (20) est configuré pour interpréter le signal de détection afin de détecter la manœuvre de Valsalva pendant une surveillance, et lorsqu'elle est détectée, pour générer des informations consultatives pour un examen clinique ultérieur.

7. Scanner médical selon l'une quelconque des revendications 1 à 5, dans lequel l'agencement de processeur (20) est configuré pour cesser la délivrance de rayonnement de balayage ou fournir des instructions à un opérateur de scanner pour mettre fin à la délivrance de rayonnement de balayage si une manœuvre de Valsalva est détectée pendant un scanner avec produit de contraste.

8. Scanner médical selon l'une quelconque des revendications 1 à 5, dans lequel l'agencement de processeur (20) est configuré pour mettre fin à l'injection de produit de contraste si une manœuvre de Valsalva est détectée pendant une apnée lors d'un scanner avec produit de contraste.

9. Scanner médical selon l'une quelconque des revendications 1 à 8, comprenant en outre un écran (40) pour présenter des informations à un sujet pendant le balayage, dans lequel l'agencement de processeur est en outre configuré pour commander l'écran afin de présenter des instructions de retenue de respiration au sujet.

10. Scanner médical selon l'une quelconque des revendications 1 à 9, dans lequel le système de scanner (12) est un système de tomodensitométrie.

11. Scanner médical selon l'une quelconque des revendications 1 à 9, dans lequel le système de scanner (12) est un système de scanner IRM.
